(19) Europäisches Patentamt
European Patent Office
Office européen des brevets

(11) Veröffentlichungsnummer: **0 004 660**
**B1**

(12) **EUROPÄISCHE PATENTSCHRIFT**

(45) Veröffentlichungstag der Patentschrift: **29.04.81**

(21) Anmeldenummer: **79101002.8**

(22) Anmeldetag: **31.03.79**

(51) Int. Cl.³: **C 07 C 120/04,
C 07 C 121/75**

# (54) Verfahren zur Herstellung von 2,5-Dichlor-4-cyanophenol.

(30) Priorität: **05.04.78 DE 2814690**

(43) Veröffentlichungstag der Anmeldung:
**17.10.79 Patentblatt 79/21**

(45) Bekanntmachung des Hinweises auf die Patenterteilung:
**29.04.81 Patentblatt 81/17**

(84) Benannte Vertragsstaaten:
**BE CH DE FR GB IT LU NL**

(56) Entgegenhaltungen:
**BE - A - 756 895**

(73) Patentinhaber: **CELAMERCK GmbH & Co. KG
Binger Strasse 173
D-6507 Ingelheim am Rhein (DE)**

(72) Erfinder: **Sehring, Richard, Dr.
Frankenstrasse 13
D-6507 Ingelheim/Rhein (DE)**
Erfinder: **Buck, Wolfgang, Dr.
In der Dörrwies 37
D-6507 Ingelheim/Rhein (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 004 660 B1

Courier Press, Leamington Spa, England.

# Verfahren zur Herstellung von 2,5-Dichlor-4-cyanophenol

Die Erfindung betrifft ein neues, vorteilhaftes Verfahren zur Herstellung des 2,5-Dichlor-4-cyanophenols. Dieses Phenol ist ein bisher schwer zugängliches Zwischenprodukt, z.B. für die Herstellung des hochwirksamen insektiziden Wirkstoffs O,O-Diäthyl-O-(2,5-dichlor-4-cyanophenyl)-thiono-phosphat. Die übliche Synthese des 2,5-Dichlor-4-cyanophenols, ausgehend von 2,5-Dichlorphenol gemäß der Sandmeyer-Reaktion, ergibt lediglich eine Gesamtausbeute von rund 10% der Theorie.

Aus der BE - PS 756 895 ist bekannt, daß durch Umsetzung des 4-Bromphenols mit Kupfer(I)cyanid bei 180—200°C in hoher Ausbeute 4-Cyanophenol entsteht. Dieses Verfahren läßt sich jedoch nicht auf 2,5-Dichlor-4-bromphenol übertragen, da es bei diesem zu beträchtlicher Verharzung und zur Bildung hochtoxischer Nebenprodukte kommt. Durch Einführung einer Schutzgruppe R für die phenolische OH-Gruppe werden störende Nebenreaktionen an den Chloratomen und an der Hydroxygruppe vermieden, während die Reaktivität des 4-ständigen Bromatoms ungeschmälert erhalten bleibt.

Das neue Verfahren ist dadurch gekennzeichnet, daß man ein Derivat des 2,5-Dichlor-4-bromphenols der Formel

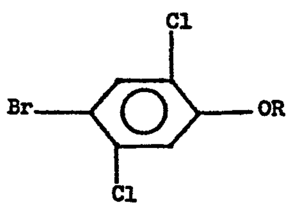

(I),

worin R für einen Acylrest, einen Alkylrest oder einen Aralkylrest steht, mit Kupfer(I)cyanid in einem polaren Lösungsmittel zu der Verbindung der Formel

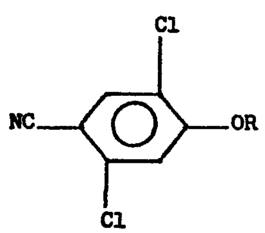

(II)

umsetzt und anschließend die Schutzgruppe R abspaltet.

Als Schutzgruppe R kommen als Acylreste beispielsweise niedere aliphatische Carbonsäurereste wie Acetyl, Propionyl, aber auch Reste anderer Carbonsäuren, z.B. Benzoyl, in Betracht. Als Alkylreste dienen vor allem niedere Alkylreste, z.B. Methyl, als araliphatischer Rest eignet sich z.B. Benzyl. Die Umsetzung der Bromverbindung I zu der Cyanoverbindung II erfolgt in bzw. in Gegenwart inerter, polarer Lösungsmittel, z.B. in Dimethylformamid, Diäthylformamid, N-Methylpyrrolidon, bei erhöhter Temperatur, vorzugsweise zwischen etwa 100°C und der Siedetemperatur des Reaktionsgemisches.

Die zweite Reaktionsstufe, die Abspaltung der Schutzgruppe, wird in an sich bekannter Weise durchgeführt. Acylgruppen werden in saurer oder basischer Lösung, insbesondere in ammoniakalischer oder wässrig-alkalischer Lösung abgespalten, bevorzugt in der Wärme. Durch Ansäuern der entstehenden Phenolatlösung wird das Phenol freigesetzt und in üblicher Weise isoliert. Ist die phenolische OH-Gruppe in Form einer Äthergruppierung geschützt, beispielsweise als Methyläther, so kann die Ätherabspaltung z.B. mit HCl/Pyridin bei erhöhter Temperatur, bevorzugt bei etwa 130—160°C, erfolgen. Anschließend wird das Phenol aus dem Reaktionsgemisch isoliert.

Die glatte Reaktion der Verbindungen der Formel I zu den entsprechenden 4-Cyanoverbindungen der Formel II war nicht vorherzusehen.

Erfindungsgemäß kann nun das 2,5-Dichlor-4-cyanophenol in so guter Ausbeute erhalten werden, daß jetzt auch eine rationelle Herstellung der Verbindung in technischem Maßstab möglich ist.

## Beispiel 1

315 g 2,5-Dichlor-4-bromphenolacetat und 90 g Kupfer(I)cyanid werden in 300 ml Dimethylformamid zunächst 1 Stunde bei 120—130°C, dann noch 2 Stunden bei 140—145°C gerührt. Man filtriert und engt am Rotationsverdampfer bei ca. 20 mbar (15 Torr) und 90—100°C zur Trockne ein. Der Rückstand wird in 500 ml Toluol heiß gelöst und filtriert, das Lösungsmittel im Vakuum abdestilliert. Der Rückstand wird in einer Mischung von 400 ml konz. wässrigem Ammoniak und 1,5 l Wasser bei 60°C verseift. Man säuert die filtrierte Lösung mit verdünnter Schwefelsäure an, rührt die erhaltenen Kristalle mit 250 ml Toluol aus, saugt ab und trocknet. 56 g Ausgangsprodukt werden zurückgewonnen. Ausbeute 160 g (85% d.Th.); Fp. 197°C.

Das 2,5-Dichlor-4-bromphenolacetat kann z.B. durch Acetylieren des Phenols mit Acetanhydrid erhalten werden.

## Beispiel 2

140 g 2,5-Dichlor-4-bromanisol und 45 g Kupfer(I)cyanid werden in 140 ml Dimethylformamid zunächst 1 Stunde bei 120—130°C, dann 4 Stunden bei etwa 140°C gerührt. Man filtriert und dampft das Filtrat am Rotationsverdampfer bei 20 mbar (15 Torr) zur Trockne ein.

Der Rückstand wird mit 80 g Pyridin versetzt und HCl-Gas eingeleitet. Dabei wird die

Temperatur langsam bis auf 150°C gesteigert. Bei dieser Temperatur wird noch 3 Stunden langsam HCl eingeleitet. Dann gießt man das Reaktionsgemisch in Eiswasser, saugt ab und trocknet das erhaltene Produkt.

Ausbeute: 66 g (70% d.Th.); Fp. 198°C (aus Toluol).

**Patentanspruch**

Verfahren zur Herstellung von 2,5-Dichlor-4-cyanophenol, dadurch gekennzeichnet, daß man ein Derivat des 2,5-Dichlor-4-bromphenols der Formel

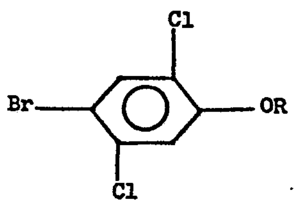

(I)

worin R für einen Acylrest, einen Alkylrest oder einen Aralkylrest steht, mit Kupfer(I)cyanid in einem polaren Lösungsmittel zu der Verbindung der Formel

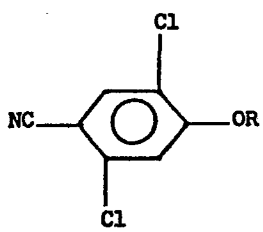

(II)

umsetzt und anschließend die Schutzgruppe R abspaltet.

**Revendication**

Procédé pour la préparation du 2,5-dichloro-4-cyanophénol, caractérisé en ce que l'on fait réagir un dérivé du 2,5-dichloro-4-bromophénol de formule:

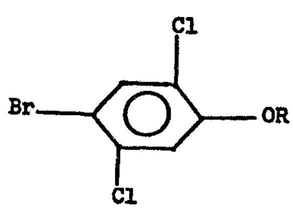

(I),

dans laquelle R représente un radical acyle, un radical alcoyle ou un radical aralcoyle, avec du cyanure de cuivre (I) dans un solvant polaire pour donner le composé de formule:

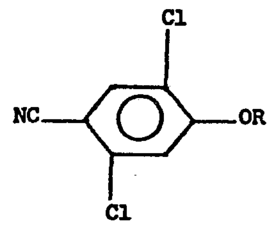

(II)

et ensuite on clive le groupe protecteur R.

**Claim**

Process for the preparation of 2,5-dichloro-4-cyanophenol, characterised in that a derivative of 2,5-dichloro-4-bromophenol of the formula

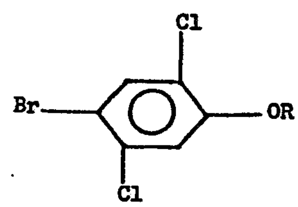

(I),

wherein R represents an acyl group, an alkyl group or an aralkyl group, is reacted with copper (I) cyanide in a polar solvent to produce a compound of formula

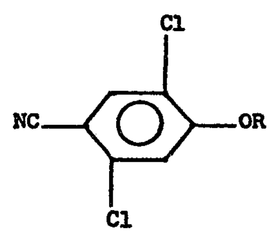

(II)

and the protecting group R is subsequently split off.